(19) Europäisches Patentamt
European Patent Office
Office européen des brevets

(11) Veröffentlichungsnummer: **0 044 993**
**B1**

(12) # EUROPÄISCHE PATENTSCHRIFT

(45) Veröffentlichungstag der Patentschrift: **29.05.85**

(51) Int. Cl.⁴: **C 07 D 249/08**, A 01 N 43/64 // C07C49/16, C07C49/327, C07C49/233

(21) Anmeldenummer: **81105442.8**

(22) Anmeldetag: **13.07.81**

(54) **Triazolylpropenol-Derivate, Verfahren zu ihrer Herstellung sowie ihre Verwendung als Pflanzenwachstumsregulatoren und Fungizide.**

(30) Priorität: **25.07.80 DE 3028330**
**20.03.81 DE 3111013**

(43) Veröffentlichungstag der Anmeldung:
**03.02.82 Patentblatt 82/5**

(45) Bekanntmachung des Hinweises auf die Patenterteilung:
**29.05.85 Patentblatt 85/22**

(84) Benannte Vertragsstaaten:
**AT BE CH DE FR GB IT LI NL SE**

(56) Entgegenhaltungen:
**DE - A - 2 838 847**
**DE - A - 3 010 560**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Regel, Erik, Ing.-grad., Bergerheide 72a, D-5600 Wuppertal 1 (DE)**
Erfinder: **Büchel, Karl Heinz, Prof. Dr., Dabringhausener Strasse 42, D-5093 Burscheid (DE)**
Erfinder: **Lürssen, Klaus, Dr., August-Kierspel-Strasse 89, D-5060 Bergisch-Gladbach 2 (DE)**
Erfinder: **Frohberger, Paul-Ernst, Dr., Willi-Baumeister-Strasse 5, D-5090 Leverkusen 1 (DE)**
Erfinder: **Paul, Volker, Dr., Ahornstrasse 5, D-5650 Solingen (DE)**

**EP 0 044 993 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue Triazolylpropenol-Derivate, ein Verfahren zu ihrer Herstellung sowie ihre Verwendung als Pflanzenwachstumsregulatoren und Fungizide.

Es ist bereits bekannt geworden, daß 4,4-Dimethyl-1-phenyl-2-triazolyl-1-penten-3-ole eine gute fungizide Wirksamkeit besitzen (vgl. DE-A 2 838 847). Die Wirkung dieser Verbindungen ist jedoch, insbesondere bei niedrigen Aufwandmengen und -konzentrationen, nicht immer voll befriedigend. Die pflanzenwachstumsregulierende Wirkung dieser Azol-Derivate ist ebenfalls nicht immer ganz befriedigend.

Außerdem sind aus der DE-A 3 010 560 bestimmte geometrische Isomere von Azol-Derivaten mit fugiziden, herbiziden und pflanzenwuchsregulierenden Eigenschaften bekannt. Es werden jedoch keine Verbindungen beschrieben, in denen ein durch Ethyl substituierter Cyclopropyl-Rest oder ein substituierter Alkylrest über eine Carbinol-Gruppe mit dem verbleibenden Molekülteil verbunden ist.

Es wurden nun neue Triazolylpropenol-Derivate der Formel

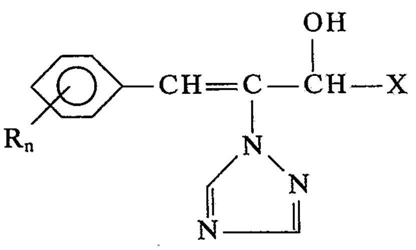

(I)

in welcher

R für Fluor oder Chlor steht,
n für 1 oder 2 steht und
X für durch Ethyl substituiertes Cyclopropyl oder für die Gruppierung

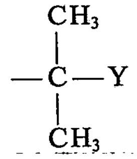

steht, in welcher

Y für gegebenenfalls durch Chlor substituiertes Phenyl, Methoxycarbonyl, Ethoxycarbonyl, Isopropoxycarbonyl, Isobutoxycarbonyl oder tert.-Butoxycarbonyl steht,

oder

R für Trifluormethoxy steht,
n für 1 steht und
X für durch Methyl substituiertes Cyclopropyl steht,

sowie deren Chlorwasserstoff- oder 1,5-Naphthalindisulfonsäure-Additionssalze gefunden.

Die erfindungsgemäßen Verbindungen der Formel (I) kommen in den geometrischen Isomeren E (trans) und Z (cis) vor.

Bei der E,Z-Nomenklatur werden die an der Doppelbindung stehenden Substituenten nach der Cahn-Ingold-Prelog-Regel nach abnehmender Priorität eingeordnet. Stehen die bevorzugten Substituenten auf derselben Seite der Doppelbindung, liegt die Konfiguration Z (abgeleitet von zusammen) vor, stehen sie auf entgegengesetzter Seite, liegt die Konfiguration E (abgeleitet von entgegen) vor.

Da außerdem ein asymmetrisches Kohlenstoffatom vorhanden ist, können die Verbindungen der Formel (I) in zwei optischen Isomerenformen vorkommen.

Die Erfindung betrifft sowohl die einzelnen Isomeren als auch die Isomerengemische.

Weiterhin wurde gefunden, daß man die Triazolylpropenol-Derivate der Formel (I) erhält, wenn man Triazolylpropenon-Derivate der Formel

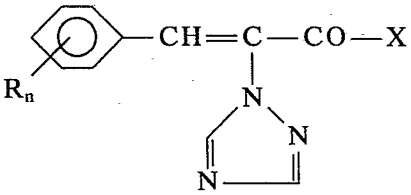

(II)

in welcher

R, X und n die oben angegebene Bedeutung haben,

in allgemein üblicher Weise reduziert.

An die so erhaltenen Verbindungen der Formel (I) kann gegebenenfalls anschließend Chlorwasserstoff oder 1,5-Naphthalindisulfonsäure addiert werden.

Schließlich wurde gefunden, daß die neuen Triazolylpropenol-Derivate der Formel (I) sowie deren Chlorwasserstoff- oder 1,5-Naphthalindisulfonsäure-Additonssalze starke pflanzenwachstumsregulierende und fungizide Eigenschaften aufweisen.

Überraschenderweise zeigen die erfindungsgemäßen Verbindungen der Formel (I) eine bessere wachstumsregulierende und fungizide Wirkung als die aus dem Stand der Technik bekannten 4,4-Dimethyl-1-phenyl-2-triazolyl-1-penten-3-ole (vgl. DE-A 2 838 847), welche chemisch und wirkungsmäßig naheliegende Verbindungen sind. Die erfindungsgemäßen Wirkstoffe stellen somit eine Bereicherung der Technik dar.

Die erfindungsgemäßen Stoffe unterscheiden sich konstitutionell eindeutig von den Azol-Derivaten, die aus der DE-A 3 010 560 bekannt sind.

Im einzelnen seien außer den bei den Herstellungsbeispielen genannten Verbindungen die folgenden Verbindungen der allgemeinen Formel (I) genannt:

Tabelle 1

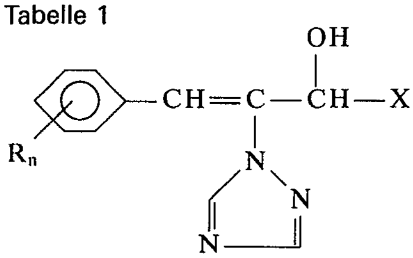

(I)

| $R_n$ | X |
|---|---|
| 4-F | $-C(CH_3)_2-$ 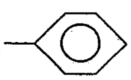 |
| 2-Cl | $-C(CH_3)_2-$ 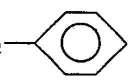 |
| 2,4-$Cl_2$ | $-C(CH_3)_2-$ 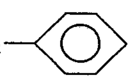 |
| 4-Cl | $-C(CH_3)_2-$ 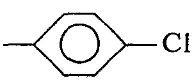 |
| 4-F | $-C(CH_3)_2-$ 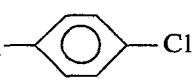 |
| 2,4-$Cl_2$ | $-C(CH_3)_2-$ 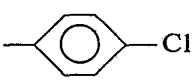 |
| 4-Cl | $-C(CH_3)_2-COOCH_3$ |
| 4-F | $-C(CH_3)_2-COOCH_3$ |
| 2-Cl | $-C(CH_3)_2-COOCH_3$ |
| 2,4-$Cl_2$ | $-C(CH_3)_2-COOCH_3$ |
| 2-Cl | $-C(CH_3)_2-COOC_2H_5$ |
| 2,4-$Cl_2$ | $-C(CH_3)_2-COOC_2H_5$ |

Verwendet man beispielsweise 1-(4-Chlorphenyl)-4-methyl-4-phenyl-2-(1,2,4-triazol-1-yl)-1-penten-3-on und Natriumborhydrid als Ausgangsstoffe, so kann der Reaktionsablauf durch das folgende Formelschema wiedergegeben werden:

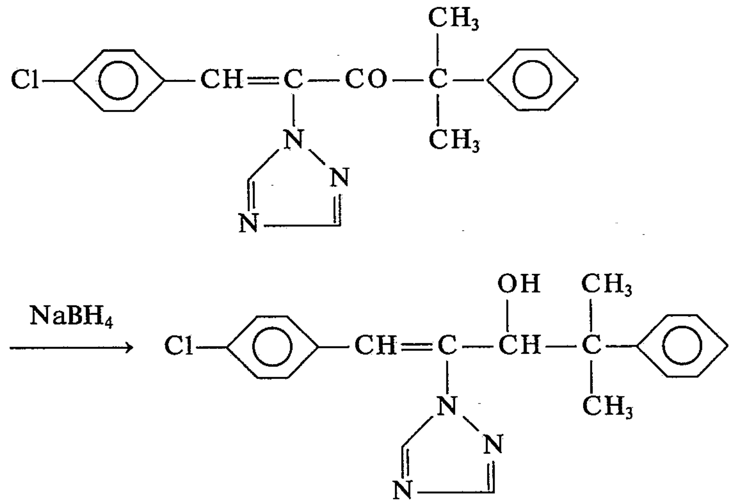


E/Z-Isomerengemisch

E/Z-Isomerengemisch

Verwendet man beispielsweise Z-isomeres 1-(4-Chlorphenyl)-4-ethoxycarbonyl-4-methyl-2-(1,2,4-triazol-1-yl)-1-penten-3-on und Aluminiumisopropylat als Ausgangsstoffe, so kann der Reaktionsablauf durch das folgende Formelschema wiedergegeben werden:

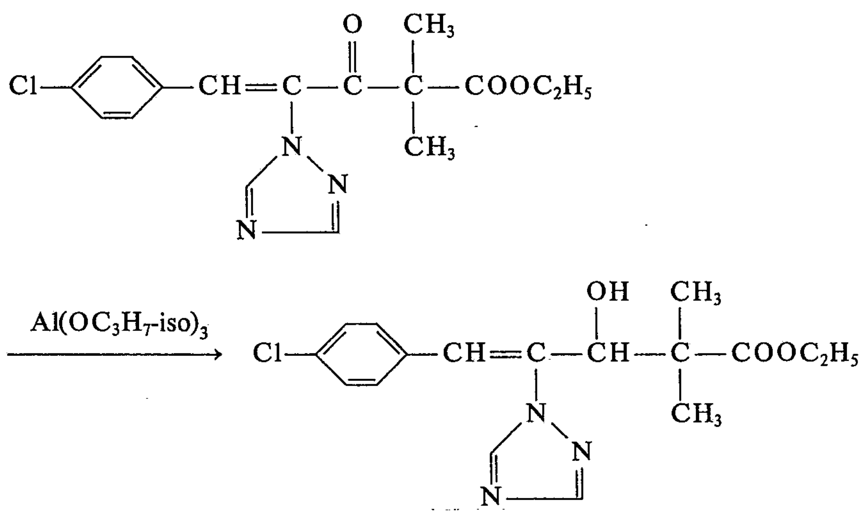


Z-Isomeres

Z-Isomeres

Die bei der Durchführung der erfindungsgemäßen Reduktion als Ausgangsstoffe benötigten Triazolylpropenon-Derivate sind durch die Formel (II) allgemein definiert. In dieser Formel haben X, R und der Index n diejenigen Bedeutungen, die bereits im Zusammenhang mit der Beschreibung der erfindungsgemäßen Stoffe der Formel (I) für diese Substituenten bzw. für diesen Index genannt wurden.

Die Triazolylpropenon-Derivate der Formel (II) sind noch nicht bekannt. Sie können jedoch in bekannter Art und Weise erhalten werden, indem man Triazolyl-ketone der Formel

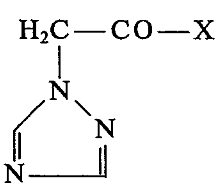

(III)

in welcher

X die oben angegebene Bedeutung hat,

mit Aldehyden der Formel

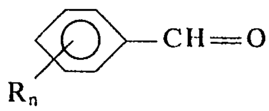

(IV)

in welcher

R und n die oben angegebene Bedeutung haben,

in Gegenwart eines Lösungsmittels und in Gegenwart eines Katalysators umsetzt.

Als Lösungsmittel kommen für die Herstellung der Triazolylpropenon-Derivate der Formel (II) vorzugsweise inerte organische Lösungsmittel in Frage. Hierzu gehören vorzugsweise Alkohole, wie Methanol und Ethanol; Ether, wie Tetrahydrofuran und Dioxan; aliphatische und cycloaliphatische Kohlenwasserstoffe, wie Hexan und Cyclohexan; aromatische Kohlenwasserstoffe, wie Benzol, Toluol und Cumol; halogenierte aliphatische und aromatische Kohlenwasserstoffe, wie Methylenchlorid, Tetrachlorkohlenstoff, Chloroform, Chlorbenzol und Dichlorbenzol.

Die Herstellung der Verbindungen der Formel (II) wird in Gegenwart eines Katalysators durchgeführt. Man kann alle üblicherweise verwendbaren sauren und insbesondere basischen Katalysatoren sowie deren Puffergemische einsetzen. Hierzu gehören vorzugsweise Lewis-Säuren, wie z. B. Bortrifluorid, Bortrichlorid, Zinntetrachlorid oder Titantetrachlorid; organische Basen wie Pyridin und Piperidin; sowie insbesondere Piperidinacetat.

Die Reaktionstemperaturen können bei der Durchführung dieses Verfahrens in einem größeren Bereich variiert werden. Im allgemeinen arbeitet man zwischen 20 und 160°C, vorzugsweise bei der Siedetemperatur des jeweiligen Lösungsmittels.

Bei der Durchführung dieses Verfahrens setzt man auf 1 Mol Triazolylketon der Formel (III) 1 bis 1,5 Mol Aldehyd der Formel (IV) und katalytische bis 0,2molare Mengen an Katalysator ein. Die Produkte der Formel (II) fallen vorzugsweise als E/Z-Isomerengemische an. Eine Trennung in die reinen Isomeren ist auf übliche Art und Weise möglich, wie z. B. durch Kristallisation oder durch chromatographische Trennverfahren.

Die Triazolylpropenon-Derivate der Formel (II) stellen allgemein interessante Zwischenprodukte dar, wie z. B. zur Herstellung der erfindungsgemäßen Verbindungen der Formel (I). In entsprechenden Konzentrationen zeigen sie auch wachstumsregulierende und fungizide Eigenschaften.

Die Triazolylketone der Formel (III) sind teilweise bekannt (vgl. DE-A 2 431 407 und DE-A 2 638 470).

Die Triazolylketone der Formel (III) können nach allgemein bekannten Verfahren erhalten werden, indem man Halogenketone der Formel

$$Hal-CH_2-CO-X \qquad (V)$$

in welcher

X die oben angegebene Bedeutung hat und
Hal für Chlor oder Brom steht,

mit 1,2,4-Triazol in Gegenwart eines Verdünnungsmittels und in Gegenwart eines Säurebindemittels umsetzt.

Als Verdünnungsmittel kommen für die Herstellung der Triazolylketone der Formel (III) vorzugsweise inerte organische Lösungsmittel in Frage. Hierzu gehören vorzugsweise Ketone, wie Aceton und Methylethylketon; Nitrile, wie Acetonitril; Ether, wie Tetrahydrofuran oder Dioxan; aromatische Kohlenwasserstoffe, wie Benzol und Toluol; Formamide, wie Dimethylformamid; und halogenierte Kohlenwasserstoffe.

Dieses Verfahren wird in Gegenwart eines Säurebinders vorgenommen. Man kann alle üblicherweise verwendbaren anorganischen oder organischen Säurebinder zugeben, wie Alkalicarbonate, beispielsweise Natriumcarbonat, Kaliumcarbonat und Natriumhydrogencarbonat; Alkalialkoholate, wie Natriummethylat oder Natriumethylat; niedere tertiäre Alkylamine, Cycloalkylamine oder Aralkylamine, wie Triethylamin, Dimethylbenzylamin oder Dimethylcyclohexylamin; oder wie Pyridin und ein entsprechender Überschuß an 1,2,4-Triazol.

Die Reaktionstemperaturen können bei der Durchführung dieses Verfahrens in einem größeren Bereich variiert werden. Im allgemeinen arbeitet man zwischen 0 bis 120°C, vorzugsweise zwischen 20 und 100°C.

Bei der Durchführung dieses Verfahrens setzt man auf 1 Mol der Verbindungen der Formel (V) vorzugsweise 1 bis 2 Mol 1,2,4-Triazol und 1 bis 2 Mol Säurebinder ein. Die Isolierung der Verbindungen der Formel (III) erfolgt in üblicher Weise.

Die Halogenketone der Formel (V) sind bekannt bzw. können sie in allgemein bekannter Art und Weise erhalten werden, indem man Verbindungen der Formel

$$H_3C-CO-X \qquad (VI)$$

in welcher

X die oben angegebene Bedeutung hat,

in Gegenwart eines inerten organischen Lösungsmittels bei Raumtemperatur mit Chlor oder Brom versetzt; oder z. B. mit üblichen Chlorierungsmitteln, wie Sulfurylchlorid bei 20 bis 60° C umsetzt.

Die außerdem für die Herstellung der Triazolylpropenon-Derivate der Formel (II) als Ausgangsstoffe benötigten Aldehyde der Formel (IV) sind allgemein bekannte Verbindungen der organischen Chemie.

Die erfindungsgemäße Reduktion erfolgt in üblicher Weise, z. B. durch Umsetzung mit komplexen Hydriden, gegebenenfalls in Gegenwart eines Verdünnungsmittels, oder durch Umsetzung mit Aluminiumisopropylat in Gegenwart eines Verdünnungsmittels. Die Ausgangsstoffe der Formel (II) können dabei als E/Z-Isomerengemische oder als reine Isomeren eingesetzt werden.

Arbeitet man mit komplexen Hydriden, so kommen als Verdünnungsmittel für die erfindungsgemäße Umsetzung polare organische Lösungsmittel in Frage. Hierzu gehören vorzugsweise Alkohole, wie Methanol, Ethanol, Butanol, Isopropanol, und Ether, wie Diethylether oder Tetrahydrofuran. Die Reaktion wird im allgemeinen bei $-10$ bis $+30°$ C, vorzugsweise bei $-10$ bis 20° C durchgeführt. Hierzu setzt man auf 1 Mol des Ketons der Formel (II) etwa 1 Mol eines komplexen Hydrids, wie Natriumborhydrid, Calciumborhydrid oder Lithiumalanat, ein. Die Isolierung der erfindungsgemäßen Verbindungen erfolgt in üblicher Weise, ebenso eine eventuelle Trennung der E/Z-Isomerengemische, die bei der Reduktion mit komplexen Hydriden immer entstehen, wenn man von E/Z-Isomerengemischen als Ausgangsprodukten der Formel (II) ausgeht.

Arbeitet man mit Aluminiumisopropylat, so kommen als Verdünnungsmittel für die erfindungsgemäße Umsetzung bevorzugt Alkohole, wie Isopropanol, oder inerte Kohlenwasserstoffe, wie Benzol, in Frage. Die Reaktionstemperaturen können wiederum in einem größeren Bereich variiert werden; im allgemeinen arbeitet man zwischen 20 und 120° C, vorzugsweise bei 50 bis 100° C. Zur Durchführung der Reaktion setzt man auf 1 Mol des entsprechenden Ketones der Formel (II) etwa 1 bis 2 Mol Aluminiumisopropylat ein. Die Isolierung der erfindungsgemäßen Verbindungen erfolgt in üblicher Weise.

Bei der Reduktion mit Aluminiumisopropylat erhält man ausschließlich die Z-Isomeren.

Ein eindeutiges Charakterisierungsmerkmal für die beiden geometrischen Isomeren ist die $H^1$-Kernresonanz der zwei Triazol-Protonen. Die Differenz der Shiftwerte für diese beiden Protonen ist in den E-Formen ungefähr doppelt so groß wie in den entsprechenden Z-Formen.

Die Chlorwasserstoff- oder 1,5-Naphthalindisulfonsäure-Additionssalze der Verbindungen der Verbindungen der Formel (I) können in einfacher Weise nach üblichen Salzbildungsmethoden, z. B. durch Lösen einer Verbindung der Formel (I) in einem geeigneten inerten Lösungsmittel und Hinzufügen der Chlorwasserstoffsäure oder der 1,5-Naphthalindisulfonsäure, erhalten werden und in bekannter Weise, z. B. durch Abfiltrieren, isoliert und gegebenenfalls durch Waschen mit einem inerten organischen Lösungsmittel gereinigt werden.

Die erfindungsgemäß verwendbaren Wirkstoffe greifen in den Metabolismus der Pflanzen ein und können deshalb als Wachstumsregulatoren eingesetzt werden.

Für die Wirkungsweise von Pflanzenwachstumsregulatoren gilt nach der bisherigen Erfahrung, daß ein Wirkstoff auch mehrere verschiedenartige Wirkungen auf Pflanzen ausüben kann. Die Wirkungen der Stoffe hängen im wesentlichen ab von dem Zeitpunkt der Anwendung bezogen auf das Entwicklungsstadium der Pflanze sowie von den auf die Pflanzen oder ihre Umgebung ausgebrachten Wirkstoffmengen und von der Art der Applikation. In jedem Fall sollen Wachstumsregulatoren die Kulturpflanzen in bestimmter gewünschter Weise beeinflussen.

Pflanzenwuchsregulierende Stoffe können z. B. zur Hemmung des vegetativen Wachstums der Pflanzen eingesetzt werden. Eine derartige Wuchshemmung ist u. a. bei Gräsern von wirtschaftlichem Interesse, denn dadurch kann die Häufigkeit der Grasschnitte in Ziergärten, Park- und Sportanlagen, an Straßenrändern, auf Flughäfen oder in Obstanlagen reduziert werden. Von Bedeutung ist auch die Hemmung des Wuchses von krautigen und holzigen Pflanzen an Straßenrändern und in der Nähe von Pipelines oder Überlandleitungen oder ganz allgemein in Bereichen, in denen ein starker Zuwachs der Pflanzen unerwünscht ist.

Wichtig ist auch die Anwendung von Wachstumsregulatoren zur Hemmung des Längenwachstums von Getreide. Hierdurch wird die Gefahr des Umknickens (»Lagerns«) der Pflanzen vor der Ernte verringert oder vollkommen beseitigt. Außerdem können Wachstumsregulatoren bei Getreide eine Halmverstärkung hervorrufen, die ebenfalls dem Lagern entgegenwirkt. Die Anwendung von Wachstumsregulatoren zur Halmverkürzung und Halmverstärkung erlaubt es, höhere Düngermengen auszubringen, um den Ertrag zu steigern, ohne daß die Gefahr besteht, daß das Getreide lagert.

Eine Hemmung des vegetativen Wachstums ermöglicht bei vielen Kulturpflanzen eine dichtere Anpflanzung, so daß Mehrerträge bezogen auf die Bodenfläche erzielt werden können. Ein Vorteil der

so erzielten kleineren Pflanzen ist auch, daß die Kultur leichter bearbeitet und beerntet werden kann.

Eine Hemmung des vegetativen Wachstums der Pflanzen kann auch dadurch zu Ertragssteigerungen führen, daß die Nährstoffe und Assimilate in stärkerem Maße der Blüten- und Fruchtbildung zugute kommen als den vegetativen Pflanzenteilen.

Mit Wachstumsregulatoren läßt sich häufig auch eine Förderung des vegetativen Wachstums erzielen. Dies ist von großem Nutzen, wenn die vegetativen Pflanzenteile geerntet werden. Eine Förderung des vegetativen Wachstums kann aber auch gleichzeitig zu einer Förderung des generativen Wachstums führen, dadurch, daß mehr Assimilate gebildet werden, so daß mehr oder größere Früchte entstehen.

Ertragssteigerungen können in manchen Fällen durch einen Eingriff in den pflanzlichen Stoffwechsel erreicht werden, ohne daß sich Änderungen des vegetativen Wachstums bemerkbar machen. Ferner kann mit Wachstumsregulatoren eine Veränderung der Zusammensetzung der Pflanzen erreicht werden, was wiederum zu einer Qualitätsverbesserung der Ernteprodukte führen kann. So ist es beispielsweise möglich, den Gehalt an Zucker in Zuckerrüben, Zuckerrohr, Ananas sowie in Zitrusfrüchten zu erhöhen oder den Proteingehalt in Soja oder Getreide zu steigern. Auch ist es beispielsweise möglich, den Abbau erwünschter Inhaltsstoffe, wie z. B. Zucker in Zuckerrüben oder Zuckerrohr, mit Wachstumsregulatoren vor oder nach der Ernte zu hemmen. Außerdem läßt sich die Produktion oder der Abfluß von sekundären Pflanzeninhaltsstoffen positiv beeinflussen. Als Beispiel sei die Stimulierung des Latexflusses bei Gummibäumen genannt.

Unter dem Einfluß von Wachstumsregulatoren kann es zur Ausbildung parthenokarper Früchte kommen. Ferner kann das Geschlecht der Blüten beeinflußt werden. Auch kann eine Sterilität des Pollens erzeugt werden, was bei der Züchtung und Herstellung von Hybridsaatgut eine große Bedeutung hat.

Durch den Einsatz von Wachstumsregulatoren läßt sich die Verzweigung der Pflanzen steuern. Einerseits kann durch Brechen der Apikaldominanz die Entwicklung von Seitentrieben gefördert werden, was besonders im Zierpflanzenbau auch in Verbindung mit einer Wuchshemmung sehr erwünscht sein kann. Andererseits ist es aber auch möglich, das Wachstum der Seitentriebe zu hemmen. Für diese Wirkung besteht z. B. großes Interesse im Tabakanbau oder bei der Anpflanzung von Tomaten.

Unter dem Einfluß von Wachstumsregulatoren kann der Blattbestand der Pflanzen so gesteuert werden, daß ein Entblättern der Pflanzen zu einem gewünschten Zeitpunkt erreicht wird. Eine derartige Entlaubung spielt bei der mechanischen Beerntung der Baumwolle eine große Rolle, ist aber auch in anderen Kulturen wie z. B. im Weinbau zur Erleichterung der Ernte von Interesse. Eine Entlaubung der Pflanzen kann auch vorgenommen werden, um die Transpiration der Pflanzen vor dem Verpflanzen herabzusetzen.

Ebenso läßt sich mit Wachstumsregulatoren der Fruchtfall steuern. Einerseits kann ein vorzeitiger Fruchtfall verhindert werden. Andererseits kann aber auch der Fruchtfall oder sogar das Abfallen der Blüten bis zu einem gewünschten Maße gefördert werden (»Ausdünnung«), um die Alternanz zu brechen. Unter Alternanz versteht man die Eigenart einiger Obstarten, endogen bedingt von Jahr zu Jahr sehr unterschiedliche Erträge zu bringen. Schließlich ist es möglich, mit Wachstumsregulatoren zum Zeitpunkt der Ernte die zum Ablösen der Früchte erforderlichen Kräfte zu reduzieren, um eine mechanische Beerntung zu ermöglichen oder eine manuelle Beerntung zu erleichtern.

Mit Wachstumsregulatoren läßt sich ferner eine Beschleunigung oder auch Verzögerung der Reife des Erntegutes vor oder nach der Ernte erreichen. Dieses ist von besonderem Vorteil, weil sich dadurch eine optimale Anpassung an die Bedürfnisse des Marktes herbeiführen läßt. Weiterhin können Wachstumsregulatoren in manchen Fällen die Fruchtausfärbung verbessern. Darüber hinaus kann mit Wachstumsregulatoren auch eine zeitliche Konzentrierung der Reife erzielt werden. Damit werden die Voraussetzungen dafür geschaffen, daß z. B. bei Tabak, Tomaten oder Kaffee eine vollständig mechanische oder manuelle Beerntung in einem Arbeitsgang vorgenommen werden kann.

Durch Anwendung von Wachstumsregulatoren kann ferner die Samen- oder Knospenruhe der Pflanzen beeinflußt werden, so daß die Pflanzen, wie z. B. Ananas oder Zierpflanzen in Gärtnereien, zu einem Zeitpunkt keimen, austreiben oder blühen, an dem sie normalerweise hierzu keine Bereitschaft zeigen. Eine Verzögerung des Austriebes von Knospen oder der Keimung von Samen mit Hilfe von Wachstumsregulatoren kann in frostgefährdeten Gebieten erwünscht sein, um Schädigungen durch Spätfröste zu vermeiden.

Schließlich kann mit Wachstumsregulatoren eine Resistenz der Pflanzen gegen Frost, Trockenheit oder hohen Salzgehalt des Bodens induziert werden. Hierdurch wird die Kultivierung von Pflanzen in Gebieten möglich, die hierzu normalerweise ungeeignet sind.

Die erfindungsgemäßen Wirkstoffe weisen auch eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz-und Saatgut und des Bodens.

Als Pflanzenschutzmittel können die erfindungsgemäßen Wirkstoffe mit besonders gutem Erfolg zur Bekämpfung solcher Pilze eingesetzt werden, die echte Mehltauerkrankungen hervorrufen, so zur Bekämpfung von Erysiphe-Arten, wie z. B. gegen den Erreger des Gersten- bzw. des Getreidemehltaus (Erysiphe graminis), oder zur Bekämpfung von Podosphaera-Arten, wie z. B. gegen den Erreger des Apfelmehltaus (Podosphaera leucotricha).

Außerdem zeigen die erfindungsgemäßen Stoffe ein breites fungizides in-vitro-Spektrum.

Die Wirkstoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z. B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z. B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z. B. Erdölfraktionen, Alkohole, wie Butanol oder Glykol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z. B. Aerosol-Treibgase, wie Halogenkohlenwasserstoffe sowie Butan, Propan, Stickstoff und Kohlendioxid. Als feste Trägerstoffe kommen in Frage: z. B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier- und/oder schaumerzeugende Mittel kommen in Frage: z. B. nichtionogene und anionische Emulgatoren, wie Polyoxyethylen-Fettsäureester, Polyoxyethylen-Fettalkoholether, z. B. Alkylarylpolyglykol-ether, Alkylsulfonate, Alkylsulfate, Arylsulfonate sowie Eiweißhydrolysate. Als Dispergiermittel kommen in Frage: z. B. Lignin-Sulfitablaugen und Methylcellulose. Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat.

Es können Farbstoffe wie anorganische Pigmente, z. B. Eisenoxid, Titanoxid, Ferrocyanblau und organische Farbstoffe, wie Alizarin-, Azo- und Metallphthalocyaninfarbstoff und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90%.

Die erfindungsgemäßen Wirkstoffe können in den Formulierungen in Mischung mit anderen Wirkstoffen vorliegen, wie Fungizide, Insektizide, Akarizide und Herbizide, sowie in Mischung mit Düngemitteln und anderen Wachstumsregulatoren.

Die Wirkstoffe können als solche, in Form ihrer Formulierungen oder der daraus bereiteten Anwendungsformen, wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z. B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäßen Verbindungen als Pflanzenwachstumsregulatoren können die Aufwandmengen in einem größeren Bereich variiert werden. Im allgemeinen verwendet man pro Hektar Bodenfläche 0,01 bis 50 kg, bevorzugt 0,05 bis 10 kg.

Beim Einsatz der erfindungsgemäßen Stoffe als Pflanzenwachstumsregulatoren gilt, daß die Anwendung in einem bevorzugten Zeitraum vorgenommen wird, dessen genaue Abgrenzung sich nach den klimatischen und vegetativen Gegebenheiten richtet.

Auch beim Einsatz der erfindungsgemäßen Stoffe als Fungizide kann die Aufwandmenge je nach Art der Applikation in einem größeren Bereich variiert werden. So liegen die Wirkstoffkonzentrationen bei der Behandlung von Pflanzenteilen in den Anwendungsformen im allgemeinen zwischen 1 und 0,0001 Gew.-%, vorzugsweise zwischen 0,5 und 0,001 Gew.-%. Bei der Saatgutbehandlung werden im allgemeinen Wirkstoffmengen von 0,001 bis 50 g je kg Saatgut, vorzugsweise 0,01 bis 10 g, benötigt. Bei Behandlung des Bodens sind Wirkstoffkonzentrationen von 0,00001 bis 0,1 Gew.-%, vorzugsweise von 0,0001 bis 0,02%, am Wirkungsort erforderlich.

## Herstellungsbeispiele

### Beispiele 1 und 2

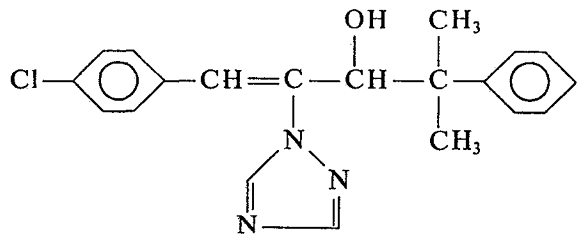

Beispiel (I-1) = Z-Isomeres

Beispiel (I-2) = E-Isomeres

16 g (0,045 Mol) 1-(4-Chlorphenyl)-4-methyl-4-phenyl-2-(1,2,4-triazol-1-yl)-1-penten-3-on als E/Z-Isomerengemisch werden in 200 ml Isopropanol gelöst und mit 0,85 g (0,0225 Mol) Natriumboranat portionsweise versetzt. Das Gemisch wird 15 Stunden bei Raumtemperatur gerührt und anschließend auf Wasser gegossen. Die organische Schicht wird mit Ether extrahiert und die vereinigten Ether-Extrakte über Natriumsulfat getrocknet. Nach Abdampfen des Ethers erhält man 14,7 g (92% der Theorie) 1-(4-Chlorphenyl)-4-methyl-4-phenyl-2-(1,2,4-triazol-1-yl)-1-penten-3-ol als E/Z-Isomerengemisch.

Durch Craig-Verteilung (Gegenstromverteilung) werden die reinen Isomeren isoliert, das Z-Isomere mit einem Schmelzpunkt von 136° C und das E-Isomere mit einem Schmelzpunkt von 140° C.

### Herstellung des Ausgangsproduktes

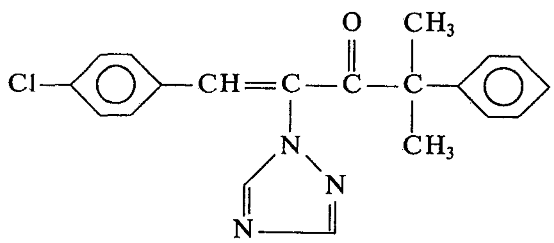 E/Z-Isomerengemisch (II-1)

40 g (0,175 Mol) 3-Methyl-3-phenyl-1-(1,2,4-triazol-1-yl)-butan-2-on und 24,5 g (0,175 Mol) 4-Chlorbenzaldehyd werden in 150 ml Toluol mit 5,25 g Eisessig und 1,75 ml Piperidin 15 Stunden unter Rückfluß erhitzt und das Reaktionswasser azeotrop abgetrennt. Die Toluollösung wird mit Wasser gewaschen, über Natriumsulfat getrocknet und im Vakuum eingedampft. Man erhält 48,6 g (79% der Theorie 1-(4-Chlorphenyl)-4-methyl-4-phenyl-2-(1,2,4-triazol-1-yl)-1-penten-3-on als E/Z-Isomerengemisch mit dem Brechungsindex $n_D^{20}$ = 1,6023.

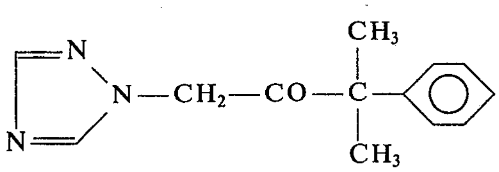 (III-1)

Zu einer Suspension von 33,4 g (0,6 Mol) Natriummethylat und 41,4 g (0,6 Mol) 1,2,4-Triazol in 200 ml Acetonitril werden 103 g (0,525 Mol) 1-Chlor-3-methyl-3-phenyl-butan-2-on getropft.

Das Reaktionsgemisch wird 18 Stunden unter Rückfluß erhitzt, kalt filtriert und das Filtrat im Vakuum eingedampft. Das resultierende Öl wird in Chloroform gelöst, mit Wasser gewaschen, über Natriumsulfat getrocknet und im Vakuum eingedampft. Man erhält 106 g (88% der Theorie) 3-Methyl-3-phenyl-1-(1,2,4-triazol-1-yl)-butan-2-on, das durch Chromatographie an Kieselgel 60 (Merck)/Chloroform gereinigt werden kann: Brechungsindex $n_D^{20}$ = 1,5425.

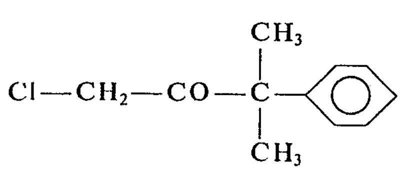

Zu einer Lösung von 16,2 g (0,1 Mol) 3-Methyl-3-phenyl-butan-2-on in 50 ml Benzol werden bei 40° C 10 ml (0,122 Mol) Sulfurylchlorid getropft. Das Gemisch wird auf 60° C gehalten, bis zur Beendigung der Gasentwicklung und anschließend destilliert. Man erhält 15,4 g (78,5% der Theorie) 1-Chlor-3-methyl-3-phenyl-butan-2-on vom Siedepunkt 80 bis 85° C/0,1 Torr bzw. vom Brechungsindex $n_D^{20}$ = 1,5310.

Beispiel 3

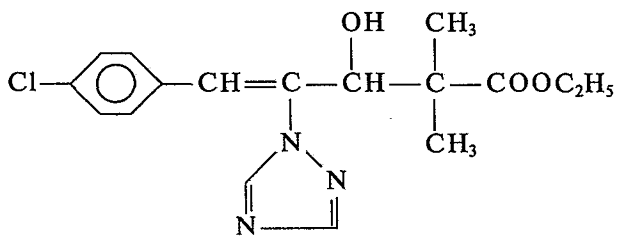
Z-Isomeres (I-3)

Ein Gemisch von 63,4 g (0,1825 Mol) Z-isomeres 1-(4-Chlorphenyl)-4-ethoxycarbonyl-4-methyl-2-(1,2,4-triazol-1-yl)-1-penten-3-on, 37,6 g (0,1825 Mol) Aluminiumisopropylat und 350 ml Isopropanol wird zum Sieden erhitzt und Aceton im Gemisch mit Isopropanol azeotrop über eine Vigreux-kolonne abdestilliert. Die Lösung wird auf eiskalte verdünnte Salzsäure gegeben und mit Methylenchlorid ausgeschüttelt. Die organische Phase wird abgetrennt, über Natriumsulfat getrocknet, filtriert und eingedampft. Das verbleibende Öl wird mit Petrolether verrührt, die erhaltenen Kristalle werden abgesaugt und mit Diisopropylether nachgewaschen. Man erhält 22,7 g (35,6% der Theorie) Z-isomeres 1-(4-Chlorphenyl)-4-ethoxycarbonyl-4-methyl-2-(1,2,4-triazol-1-yl)-1-penten-3-ol vom Schmelzpunkt 90° C.

Herstellung des Ausgangsproduktes

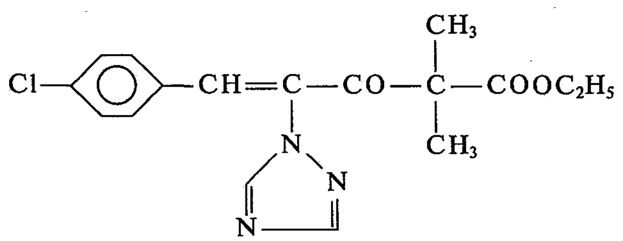
Z-Isomeres (II-2)

Ein Gemisch von 90 g (0,4 Mol) Dimethyl-(1,2,4-triazol-1-yl-acetyl)-essigsäureethylester, 56,2 g (0,4 Mol) 4-Chlorbenzaldehyd, 12 g Essigsäure und 5 ml 2,6-Dimethylmorpholin in 300 ml Toluol wird 18 Stunden unter Rückfluß erhitzt und das Reaktionswasser kontinuierlich abgetrennt. Die erkaltete Reaktionslösung wird mit Wasser gewaschen, über Natriumsulfat getrocknet, filtriert und eingeengt. Man erhält 124 g (90% der Theorie) 1-(4-Chlorphenyl)-4-ethoxycarbonyl-4-methyl-2-(1,2,4-triazol-1-yl)-1-penten-3-on vom Brechungsindex $n_D^{20}$ = 1,5751.

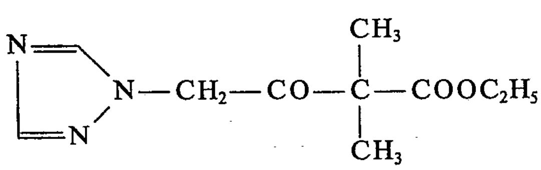
(III-2)

Zu einer Suspension von 96,7 g (1,4 Mol) 1,2,4-Triazol und 144,9 g (1,05 Mol) Kaliumcarbonat in 1200 ml Aceton werden bei 25° C 165,9 g (0,7 Mol) Bromacetyl-dimethyl-essigsäureethylester eingetropft. Nach 15stündigem Rühren werden die Salze abfiltriert und das Filtrat eingedampft. Das verbleibende Öl wird über Kieselgel-60 (Merck) gereinigt. Man erhält 120,7 g (76,6% der Theorie) Dimethyl-(1,2,4-triazol-1-yl-acetyl)-essigsäureethylester vom Brechungsindex $n_D^{20}$ = 1,4770.

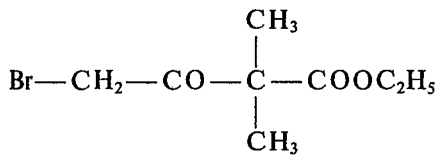

Zu einer Lösung von 295,5 g (1,87 Mol) Acetyl-dimethyl-essigsäureethylester in 1000 ml Methylalkohol werden bei 0°C 95,4 ml (1,87 Mol) Brom, gelöst in 1000 ml Chloroform, getropft. Nach 15 Stunden Rühren bei Raumtemperatur wird das Gemisch auf Eis gegeben, die Chloroformphase abgetrennt, mit Wasser gewaschen, über Natriumsulfat getrocknet, eingedampft und destilliert. Man erhält 780 g (88% der Theorie) Bromacetyl-dimethyl-essigsäureethylester vom Siedepunkt 90 bis 115°C/0,5 Torr, bzw. vom Brechungsindex $n_D^{20} = 1,4658$.

In entsprechender Weise werden die folgenden Verbindungen der allgemeinen Formel (I) erhalten:

Tabelle 2

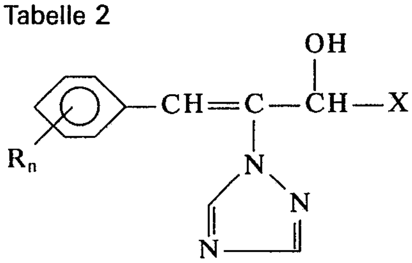

(I)

| Beispiel Nr. | $R_n$ | X | Schmelzpunkt (°C) |
|---|---|---|---|
| I-4 | 4-F | $—C(CH_3)_2—COOC_2H_5$ | 80 (Z-Isomeres) |
| I-5 | 4-$OCF_3$ | 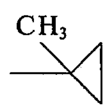 | 104 (Z-Isomeres) |
| I-6 | 4-$OCF_3$ | 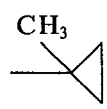 | 94 (E-Isomeres) |
| I-7 | 2,4-$Cl_2$ | 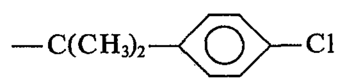 | 152 (Z-Isomeres) |
| I-8 | 2,4-$Cl_2$ | $—C(CH_3)_2—COOC_2H_5$ | 100 (Z-Isomeres) |
| I-9 | 2,4-$Cl_2$ | 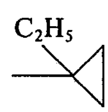 | 126 (Z-Isomeres) |
| I-10 | 2,4-$Cl_2$ | 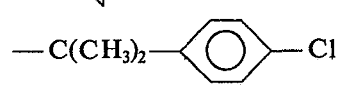 | 158 (Z-Isomeres) |
| I-11 | 2,4-$Cl_2$ | 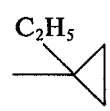 | 126 (E-Isomeres) |

Entsprechend den Herstellungsbeispielen werden die folgenden Ausgangsstoffe der Formel (II) erhalten:

Tabelle 3

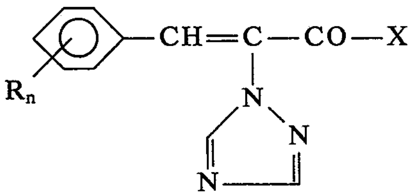

(II)

| Beispiel Nr. | $R_n$ | X | Schmelzpunkt (°C) bzw. Brechungsindex ($n_D^{20}$) |
|---|---|---|---|
| II-3 | 4-$OCF_3$ | 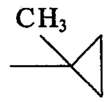 | 1,5334 (E-Isomeres) |
| II-4 | 4-$OCF_3$ | 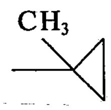 | 1,5362 (E/Z-Gemisch) |

Herstellung des Ausgangsproduktes der Formel

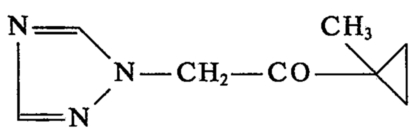

(III-3)

In eine Suspension von 27,6 g (0,4 Mol) 1,2,4-Triazol und 41,4 g (0,3 Mol) Kaliumcarbonat in 500 ml Aceton werden bei 60° C 42,5 g (0,24 Mol) 1-Bromacetyl-1-methylcyclopropan eingetropft. Nach 15 Stunden Erhitzen auf 60° C werden die Salze abgesaugt und das Filtrat im Vakuum eingedampft. Das verbleibende Öl wird chromatographisch (Kieselgel-60 (Merck)/Chloroform) gereinigt.

Man erhält 35,7 g (90% der Theorie) 1-Methyl-1-(1,2,4-triazol-1-yl-acetyl)-cyclopropan vom Schmelzpunkt 58° C.

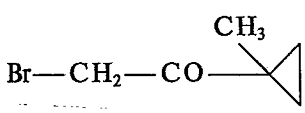

Zu einer Lösung von 29,4 g (0,3 Mol) 1-Acetyl-1-methylcyclopropan in 150 ml Methylalkohol werden bei 5° C 15 ml Brom, gelöst in 75 ml Chloroform, getropft.

Die Lösung wird bei 10° C bis zur vollständigen Entfärbung gerührt, auf Eis gegeben und mit Wasser gewaschen. Die Chloroformphase wird über Natriumsulfat getrocknet, filtriert, eingedampft und destilliert.

Man erhält 44 g (82,5% der Theorie) 1-Bromacetyl-1-methylcyclopropan vom Siedepunkt 85 bis 90° C/11 Torr bzw. vom Brechungsindex $n_D^{20}$ = 1,5002.

Entsprechend den Herstellungsbeispielen werden die folgenden Vorprodukte der Formel (III) erhalten:

Tabelle 4

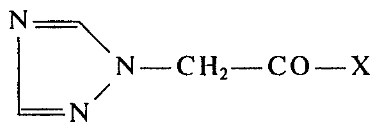
(III)

| Beispiel Nr. | X | Schmelzpunkt (°C) bzw. Brechungsindex ($n_D^{20}$) |
|---|---|---|
| III-4 | 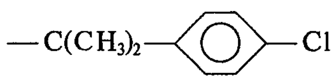 | 60°C |

## Anwendungsbeispiele

In den nachfolgenden Anwendungsbeispielen werden die nachstehend angegebenen Verbindungen als Vergleichssubstanzen eingesetzt:

(A) = 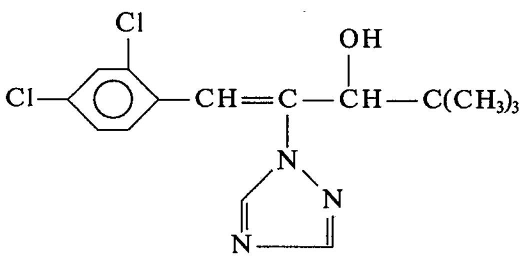 (bekannt aus DE-A 2 838 847)

(B) = 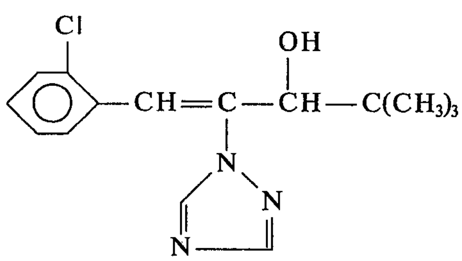

(C) = 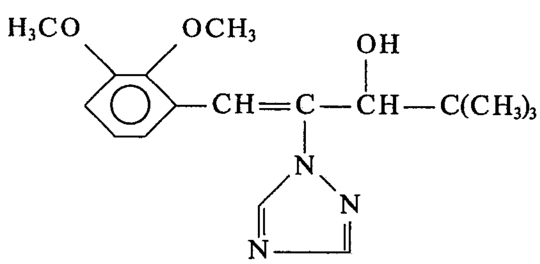

(D) = 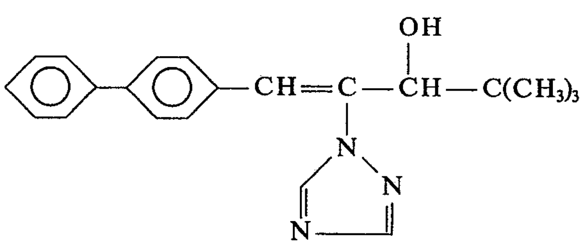 (bekannt aus DE-A 2 838 847)

## Beispiel A

### Wuchshemmung bei Baumwolle

Lösungsmittel: 30 Gew.-Teile Dimethylformamid
Emulgator: 1 Gew.-Teil Polyoxyethylen-Sorbitan-Monolaurat

Zur Herstellung einer zweckmäßigen Wirkstoffzubereitung vermischt man 1 Gew.-Teil Wirkstoff mit der angegebenen Menge Lösungsmittel und Emulgator und füllt mit Wasser auf die gewünschte Konzentration auf.

Baumwollpflanzen werden im Gewächshaus bis zur vollen Entfaltung des 5. Folgeblattes angezogen. In diesem Stadium werden die Pflanzen tropfnaß mit den Wirkstoffzubereitungen besprüht. Nach 3 Wochen wird der Zuwachs der Pflanzen gemessen und die Wuchshemmung in Prozent des Zuwachses der Kontrollpflanzen berechnet. Es bedeuten 100% Wuchshemmung den Stillstand des Wachstums und 0% ein Wachstum entsprechend dem der Kontrollpflanzen.

Der erfindungsgemäße Wirkstoff (I-11) zeigt in diesem Test eine bessere Wuchshemmung als die aus dem Stand der Technik bekannten Verbindungen (A), (B), (C) und (D).

## Beispiel B

### Wuchshemmung bei Sojabohnen

Lösungsmittel: 10 Gew.-Teile Methanol
Emulgator: 2 Gew.-Teile Polyethylen-Sorbitan-Monolaurat

Zur Herstellung einer zweckmäßigen Wirkstoffzubereitung vermischt man 1 Gew.-Teil Wirkstoff mit den angegebenen Mengen Lösungsmittel und Emulgator und füllt mit Wasser auf die gewünschte Konzentration auf.

Junge Sojabohnenpflanzen werden in dem Stadium, in dem die ersten Folgeblätter entfaltet sind, mit den Wirkstoffzubereitungen tropfnaß besprüht. Nach 2 Wochen wird der Zuwachs gemessen und die Wuchshemmung in % des Zuwachses der Kontrollpflanzen berechnet. Es bedeuten 100% den Stillstand des Wachstums und 0% ein Wachstum entsprechend dem der unbehandelten Kontrollpflanzen.

Der erfindungsgemäße Wirkstoff (I-11) zeigt in diesem Test eine bessere Wuchshemmung als die aus dem Stand der Technik bekannten Verbindungen (B), (C) und (D).

## Beispiel C

### Wuchsbeeinflussung bei Zuckerrüben

Lösungsmittel: 30 Gew.-Teile Dimethylformamid
Emulgator: 1 Gew.-Teil Polyoxyethylen-Sorbitan-Monolaurat

Zur Herstellung einer zweckmäßigen Wirkstoffzubereitung vermischt man 1 Gew.-Teil Wirkstoff mit den angegebenen Mengen Lösungsmittel und Emulgator und füllt mit Wasser auf die gewünschte Konzentration auf.

Zuckerrüben werden im Gewächshaus bis zur vollen Ausbildung der Keimblätter angezogen. In diesem Stadium werden die Pflanzen tropfnaß mit der Wirkstoffzubereitung besprüht. Nach 14 Tagen wird der Zuwachs der Pflanzen gemessen und die Wuchsbeeinflussung in Prozent des Zuwachses der Kontrollpflanzen berechnet. Es bedeuten 0% Wuchsbeeinflussung ein Wachstum entsprechend dem der Kontrollpflanzen. Negative Werte kennzeichnen eine Wuchshemmung, positive Werte eine Wuchsförderung gegenüber den Kontrollpflanzen.

Der erfindungsgemäße Wirkstoff (I-11) zeigt in diesem Test eine bessere Wuchsbeeinflussung als die aus dem Stand der Technik bekannten Verbindungen (C) und (D).

**Patentansprüche**

1. Triazolylpropenol-Derivate der Formel

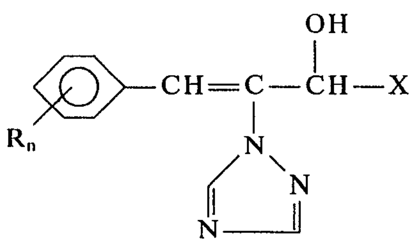

(I)

in welcher

R für Fluor oder Chlor steht,
n für 1 oder 2 steht und
X für durch Ethyl substituiertes Cyclopropyl oder für die Gruppierung

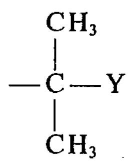

steht, in welcher

Y für gegebenenfalls durch Chlor substituiertes Phenyl, Methoxycarbonyl, Ethoxycarbonyl, Isopropoxycarbonyl, Isobutoxycarbonyl oder tert.-Butoxycarbonyl steht,

oder

R für Trifluormethoxy steht,
n für 1 steht und
X für durch Methyl substituiertes Cyclopropyl steht,

sowie deren Chlorwasserstoff- oder 1,5-Naphthalindisulfonsäure-Additionssalze.

2. Verfahren zur Herstellung von Triazolylpropenol-Derivaten der Formel

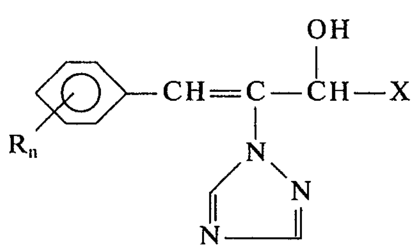

(I)

in welcher

R für Fluor oder Chlor steht,
n für 1 oder 2 steht und
X für durch Ethyl substituiertes Cyclopropyl oder für die Gruppierung

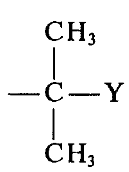

steht, in welcher

Y für gegebenenfalls durch Chlor substituiertes Phenyl, Methoxycarbonyl, Ethoxycarbonyl, Isopropoxycarbonyl, Isobutoxycarbonyl oder tert.-Butoxycarbonyl steht,

oder

R für Trifluormethoxy steht,
n für 1 steht und
X für durch Methyl substituiertes Cyclopropyl steht,

sowie von deren Chlorwasserstoff- oder 1,5-Naphthalindisulfonsäure-Additionssalzen, dadurch gekennzeichnet, daß man Triazolylpropenon-Derivate der Formel

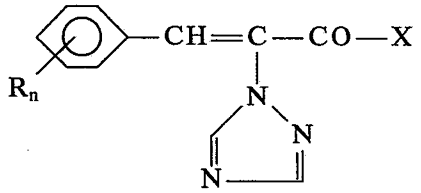

(II)

in welcher

R, X und n die oben angegebene Bedeutung haben,

in allgemein üblicher Weise reduziert, und an die so erhaltenen Verbindungen der Formel (I) noch gegebenenfalls anschließend Chlorwasserstoff oder 1,5-Naphthalindisulfonsäure addiert.

3. Pflanzenwachstumsregulierende und fungizide Mittel, gekennzeichnet durch einen Gehalt an mindestens einem Triazolylpropenol-Derivat der Formel (I) bzw. einem Chlorwasserstoff- oder 1,5-Naphthalindisulfonsäure-Additionssalz eines Triazolylpropenol-Derivates der Formel (I).

4. Verfahren zur Regulierung des Pflanzenwachstums und zur Bekämpfung von Pilzen, dadurch gekennzeichnet, daß man Triazolylpropenol-Derivate der Formel (I) bzw. deren Chlorwasserstoff- oder 1,5-Naphthalindisulfonsäure-Additionssalze auf die zu behandelnden Pflanzen oder ihren Lebensraum einwirken läßt.

5. Verwendung von Triazolylpropenol-Derivaten der Formel (I) bzw. deren Chlorwasserstoff- oder 1,5-Naphthalindisulfonsäure-Additionssalzen zur Regulierung des Pflanzenwachstums und zur Bekämpfung von Pilzen.

6. Verfahren zur Herstellung von pflanzenwachstumsregulierenden und fungiziden Mitteln, dadurch gekennzeichnet, daß man Triazolylpropenol-Derivate der Formel (I) bzw. deren Chlorwasserstoff- oder 1,5-Naphthalindisulfonsäure-Additionssalze mit Streckmitteln und/oder oberflächenaktiven Stoffen vermischt.

7. Triazolylpropenon-Derivate der Formel

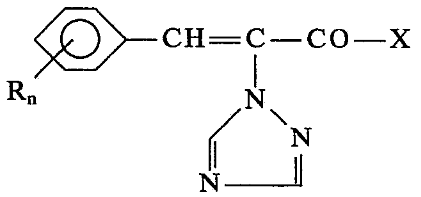

(II)

in welcher

R für Fluor oder Chlor steht,
n für 1 oder 2 steht und
X für durch Ethyl substituiertes Cyclopropyl oder für die Gruppierung

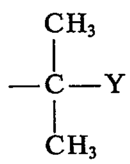

steht, in welcher

Y für gegebenenfalls durch Chlor substituiertes Phenyl, Methoxycarbonyl, Ethoxycarbonyl, Isopropoxycarbonyl, Isobutoxycarbonyl oder tert.-Butoxycarbonyl steht,

oder

R für Trifluormethoxy steht,
n für 1 steht und
X für durch Methyl substituiertes Cyclopropyl steht.

**Claims**

1. Triazolylpropenol derivatives of the formula

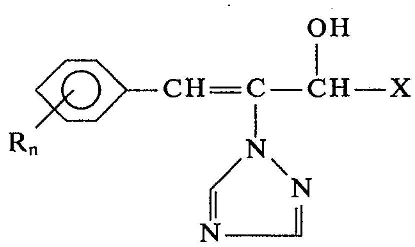

(I)

in which

R represents fluorine or chlorine,
n represents 1 or 2 and
X represents ethyl-substituted cyclopropyl or the grouping

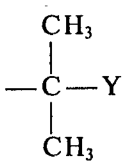

in which

Y represents optionally chlorine-substituted phenyl, methoxycarbonyl, ethoxycarbonyl, isopropoxycarbonyl, isobutoxycarbonyl or tert.-butoxycarbonyl

or

R represents trifluoromethoxy,
n represents 1 and
X represents methyl-substituted cyclopropyl,

and hydrochloric or 1,5-naphthalenedisulphonic acid addition salts thereof.

2. Process for the preparation of triazolylpropenol derivatives of the formula

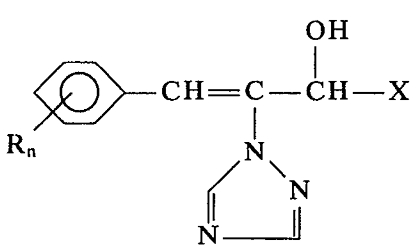

(I)

in which

R represents fluorine or chlorine,
n represents 1 or 2 and
X represents ethyl-substituted cyclopropyl or the grouping

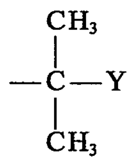

in which

Y represents optionally chlorine-substituted phenyl, methoxycarbonyl, ethoxycarbonyl, isopropoxycarbonyl, isobutoxycarbonyl or tert.-butoxycarbonyl,

or

R represents trifluoromethoxy,
n represents 1 and
X represents methyl-substituted cyclopropyl,

and hydrochloric or 1,5-naphthalenedisulphonic acid addition salts thereof, characterised in that triazolylpropenone derivatives of the formula

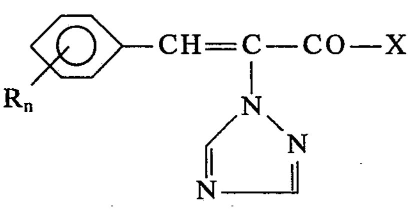

(II)

in which

R, X and n have the meaning given above,

are reduced in a generally customary manner, and hydrogen chloride or 1,5-naphthalenedisulphonic acid are then, if desired, added on to the compounds of the formula (I) thus obtained.

3. Plant-growth-regulating and fungicidal agents, characterised in that they contain at least one triazolylpropenol derivative of the formula (I) or at least one hydrochloric or 1,5-naphthalenedisulphonic acid addition salt of a triazolylpropenol derivative of the formula (I).

4. A method of regulating plant growth and for combating fungi, characterised in that triazolylpropenol derivatives of the formula (I) or hydrochloric or 1,5-naphthalenedisulphonic acid addition salts thereof are allowed to act on the plants to be treated or their habitat.

5. Use of triazolylpropenol derivatives of the formula (I) or hydrochloric or 1,5-naphthalenedisulphonic acid addition salts thereof for regulating plant growth and for combating fungi.

6. Process for the preparation of plant-growth-regulating and fungicidal agents, characterised in that triazolylpropenol derivatives of the formula (I) or hydrochloric or 1,5-naphthalenedisulphonic acid addition salts thereof are mixed with extenders and/or surface-active substances.

7. Triazolylpropenone derivatives of the formula

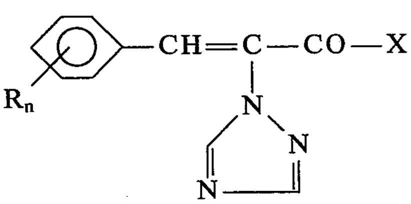

(II)

in which

R represents fluorine or chlorine,
n represents 1 or 2 and
X represents ethyl-substituted cyclopropyl or the grouping

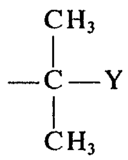

in which

Y represents optionally chlorine-substituted phenyl, methoxycarbonyl, ethoxycarbonyl, isopropoxycarbonyl, isobutoxycarbonyl or tert.-butoxycarbonyl,

or

R represents trifluoromethoxy,
n represents 1 and
X represents methyl-substituted cyclopropyl.

**Revendications**

1. Dérivés de triazolylpropénol de formule:

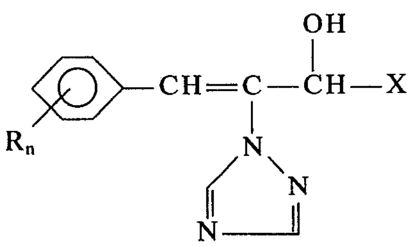

(I)

dans laquelle

R représente un atome de fluor ou un atome de chlore,
n est égal à 1 ou 2, et
X représente un groupe cyclopropyle substitué par le groupe éthyle, ou le groupement

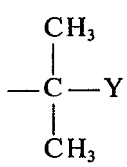

où

Y représente un groupe tert-butoxycarbonyle, un groupe isobutoxycarbonyle, un groupe isopropoxycarbonyle, un groupe éthoxycarbonyle, un groupe méthoxycarbonyle ou un groupe phényle éventuellement substitué par un atome de chlore,

ou

R représente un groupe trifluorométhoxy,
n est égal à 1, et
X représente un groupe cyclopropyle substitué par le groupe méthyle,

ainsi que leurs sels d'addition d'acide chlorhydrique ou d'acide 1,5-naphtalène-disulfonique.

2. Procédé de préparation de dérivés de triazolylpropénol de formule:

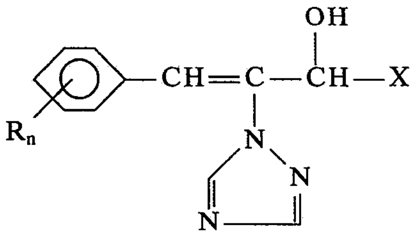

(I)

dans laquelle

R représente un atome de fluor ou un atome de chlore,
n est égal à 1 ou 2, et
X représente un groupe cyclopropyle substitué par le groupe éthyle, ou le groupement

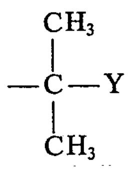

où

Y représente un groupe tert-butoxycarbonyle, un groupe isobutoxycarbonyle, un groupe isopropoxycarbonyle, un groupe éthoxycarbonyle, un groupe méthoxycarbonyle ou un groupe phényle éventuellement substitué par un atome de chlore,

ou

R représente un groupe trifluorométhoxy,
n est égal à 1, et
X représente un groupe cyclopropyle substitué par le groupe méthyle,

ainsi que de leurs sels d'addition d'acide chlorhydrique ou d'acide 1,5-naphtalène-disulfonique, caractérisé en ce qu'on réduit, de la manière généralement habituelle, des dérivés de triazolylpropénonone de formule:

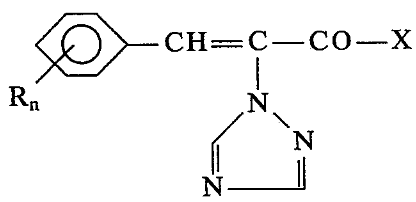

(II)

dans laquell

R, X et n ont les significations indiquées ci-dessus,

et, aux composés ainsi obtenus de formule (I), on ajoute encore éventuellement ensuite de l'acide chlorhydrique ou de l'acide 1,5-naphtalène-disulfonique.

3. Agents fongicides et régulateurs de la croissance des plantes, caractérisés en ce qu'ils contiennent au moins un dérivé de triazolylpropénol de formule (I) ou un sel d'addition d'acide chlorhydrique ou d'acide 1,5-naphtalène-disulfonique d'un dérivé de triazolylpropénol de formule (I).

4. Procédé en vue de régler la croissance des plantes et de combattre les champignons, caractérisé en ce qu'on fait agir des dérivés de triazolylpropénol de formule (I) ou leurs sels d'addition d'acide chlorhydrique ou d'acide 1,5-naphtalène-disulfonique sur les plantes à traiter ou leur biotope.

5. Utilisation de dérivés de triazolylpropénol de formule (I) ou de leurs sels d'addition d'acide chlorhydrique ou d'acide 1,5-naphtalène-disulfonique en vue de régler la croissance des plantes et de combattre les champignons.

6. Procédé de préparation d'agents fongicides et régulateurs de la croissance des plantes, caractérisé en ce qu'on mélange des dérivés de triazolylpropénol de formule (I) ou leurs sels d'addition

d'acide chlorhydrique ou d'acide 1,5-naphtalène-disulfonique avec des diluants et/ou des substances tensio-actives.

7 Dérivés de triazolylpropénone de formule:

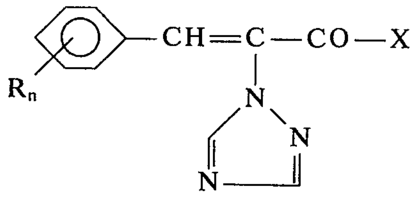

(II)

dans laquelle

R représente un atome de fluor ou un atome de chlore,
n est égal à 1 ou 2, et
X représente un groupe cyclopropyle substitué par le groupe éthyle, ou le groupement

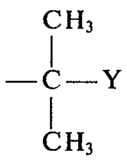

où

Y représente un groupe tert-butoxycarbonyle, un groupe isobutoxycarbonyle, un groupe isopropoxycarbonyle, un groupe éthoxycarbonyle, un groupe méthoxycarbonyle ou un groupe phényle éventuellement substitué par un atome de chlore,

ou

R représente un groupe trifluorométhoxy,
n est égal à 1 et
X représente un groupe cyclopropyle substitué par le groupe méthyle.